(19) Europäisches Patentamt
European Patent Office
Office européen des brevets

(11) EP 4 105 658 A1

(12) EUROPEAN PATENT APPLICATION

(43) Date of publication:
**21.12.2022 Bulletin 2022/51**

(21) Application number: **22160038.0**

(22) Date of filing: **03.03.2022**

(51) International Patent Classification (IPC):
***G01N 33/68*** *(2006.01)*

(52) Cooperative Patent Classification (CPC):
**G01N 33/6872; G01N 33/5008;** G01N 2333/726; G01N 2500/10

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.03.2021 IN 202121009004**

(71) Applicant: **Kashiv Biosciences, LLC**
**Piscataway, NJ 08854 (US)**

(72) Inventors:
- **Sangle, Ganesh Vishwanath**
  **443308 Dusarbid, Dist - Buldhana (IN)**
- **UNADKAT, Vishal Bharatbhai**
  **380058 Ahmedabad (IN)**
- **PANDYA, Heta Nishil**
  **380015 Ahmedabad (IN)**
- **JASH, Kavya Siddhartha**
  **380015 Ahmedabad (IN)**

(74) Representative: **Basra, Sandeep**
**Haseltine Lake LLP**
**Bürkleinstrasse 10**
**80538 München (DE)**

Remarks:
Claims: 16, 17, 19, 21-24, 26, 28-30 are deemed to be abandoned due to non-payment of the claims fees (Rule 45(3) EPC).

# (54) IMPROVED BIOASSAY METHOD

(57) The present invention relates to a determination of antagonist effect of desired compound useful in the inhibition of activity of bradykinin receptors. Furthermore, the present invention provides an improved assay method to determine the efficacy of desired compounds by inhibiting the activity of bradykinin receptors. More specifically the present invention provides an improved assay method, wherein desired antagonistic effect of the compounds is determined by measuring intracellular calcium release.

EP 4 105 658 A1

## Description

### FIELD OF THE INVENTION

The present invention relates to a determination of antagonist effect of desired compound by inhibiting the activity of receptor of interest. In particular aspect the invention provides a washing post addition of dye in the assay method which improve signal to noise ration and reduce variation thereby determine the antagonistic or agonistic effect of compound.

### BACKGROUND OF THE INVENTION

The present invention relates to a determination of antagonist effect of desired compound useful in the inhibition the activity of receptors expressed in mammalian cell. The antagonistic effect of the desired compound is determined by measuring intracellular calcium release.

The reproducibility of data is important while screening compound by assay method. It is very difficult and time consuming for skilled person to arrive at the desire results of the tested compound in absence of robust and reproducible assay method. skilled person may confuse or discard the potential compound due to variability in test result. Hence the method optimization is required to reduce assay variation.

Bradykinins (BK) are naturally occurring vasoactive peptide hormones, which are known to be important mediators of a variety of biological effects, including cardiovascular homeostasis, inflammation, and nociception. The kinins interact with two G-protein-coupled receptors, termed B1 and B2 receptors (B1R and B2R). Overproduction of kinins under pathophysiological conditions is implicated in several disorders, including pain, inflammation, hypotension, asthma, colitis, pancreatitis, rhinitis, sepsis, and rheumatoid arthritis. Therefore, antagonists of BK receptors may offer a novel approach to the treatment of these disorders, among which perhaps the most promising area is in the treatment of pain.

Pain is a complex multidimensional concept that facilitates the initiation of the signalling cascade in response to any noxious stimuli. Numerous types of receptors are activated in pain sensations which vary in their signalling pathway. These signalling pathways can be regarded as a site for modulation of pain by targeting the pain transduction molecules to produce analgesia out of several pain syndromes.

The pathogenesis of Interstitial Cystitis/Bladder Pain Syndrome (IC/BPS), over active bladder (OAB) is not clearly known and often have comorbidities of other chronic pelvic pain conditions, such as IBS and endometriosis, as well as systemic pain conditions. Peripheral inflammation produces multiple inflammatory mediators, such as bradykinin, prostaglandins (PGE2), purines (ATP), proteases and Nerve growth factor (NGF) that act on their cognate receptors expressed majorly in nociceptive sensory neurons to activate intracellular signal transduction pathways.

Therefore, blocking bradykinin receptor has pharmacological importance to treat various diseases associated with inflammation. Thus, the present invention provides an improved assay method to determine the antagonist effect and efficacy of desired compound useful in the inhibition of bradykinin receptors more specifically by measuring intracellular calcium release. The present invention provides a method to determine antagonistic effect for inhibiting bradykinin receptor which is just an example, and no way invention should be considered limiting to antagonist of bradykinin only. The skilled person in the art will predict and extrapolate the use of the present assay method for determining inhibiting activity of GPCR useful for treating diseases.

### SUMMARY OF THE INVENTION

In an embodiment, the present invention provides an improved assay method to determine the efficacy of an antagonist by inhibiting the activity of receptor of interest by performing the washing post addition of dye in the assay plate.

In an embodiment, the present invention provides an improved assay method to determine the efficacy of an antagonist by inhibiting the activity of receptor of interest, the assay method comprising:

a. seeding a cell expressing the receptor of interest on the plate;
b. optionally incubating the cell for suitable time;
c. washing the cell with suitable buffer selected from Hanks' Balanced Salt Solution (HBSS) and phosphate buffer; said buffer further comprising 1 M HEPES and optionally with probenecid;
d. mixing the suitable dye to stain the cell;
e. incubating the cell for suitable time;
f. acclimatize the cell to suitable temperature for suitable time;
g. washing the cell with suitable buffer;
h. mixing the suitable antagonist diluted in suitable buffer;
i. incubating and shaking the plate for suitable time at suitable temperature;

j. reading the plate to determine the baseline signal;
k. mixing a suitable agonist diluted in suitable buffer;
l. shaking the plate for suitable time;
m. reading the plate to determine the inhibition efficacy of antagonist;
n. Wherein the assay improved the signal to noise ratio and lower the variability, in comparison to assay performed without washing.

In one embodiment, after performing the above method the IC50 values of antagonist selected from about 1 nM to about 20 μM.
In one embodiment, current method comprises the washing step.
In one embodiment, washing improves the efficacy of assay by removing background noise.
In one embodiment, washing surprisingly improving the response of agonist and antagonist.
In one embodiment, washing reduces the variation withing the group of same concentration of compound. In other embodiment, washing reduces the variation between the replicates of same concentration of compound.
In one embodiment, the washing improves the difference between the vehicle control group and the group containing suitable compound.
In one embodiment, washing reduces the variation withing the group of same concentration for compound selected from compound I, compound II, compound III, compound IV, compound V.
In one embodiment, the present invention shows that by incorporating washing step in the protocol, higher values of fluorescence obtained which makes the window broader and hence helps to determine percent inhibition by the compounds accurately.
In one embodiment, the washing provides the higher Z' values.
In embodiment the suitable dye is selected from FLIPR Calcium 4, FLIPR Calcium 5, FLIPR Calcium 6 and 6-QF, Fluo-8 AM, fluo-4 NW, Fluo-4AM
In one embodiment, suitable dye comprising Ex/Em = about 333nm to about 665nm
In one embodiment, suitable dye comprising Ex/Em = about 494nm to about 516nm.
In another embodiment, the assay method comprises a one or more antagonist selected from the group consisting of;

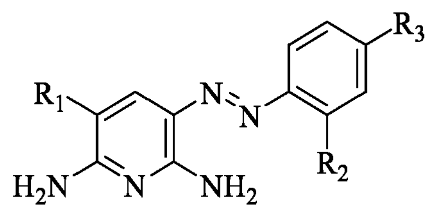

Formula I

; and pharmaceutically acceptable salts, solvates thereof; wherein $R_1$, $R_2$ and $R_3$ can be selected independently from hydrogen and hydroxyl group.
In another embodiment, the assay method comprises a one or more antagonist selected from the group consisting of;

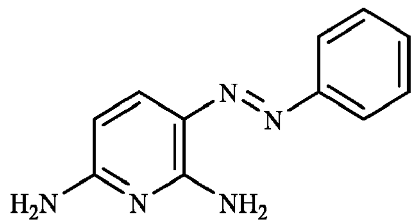

Compound I

,

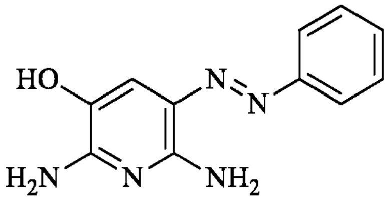

Compound II ,

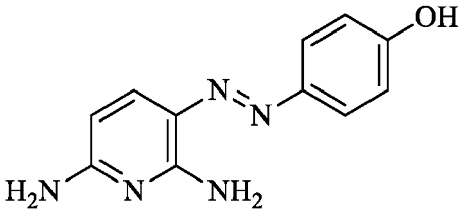

Compound III ,

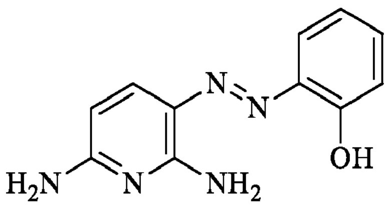

Compound IV ,

and

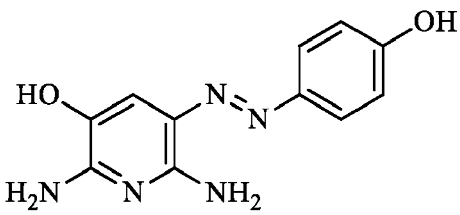

Compound V ;

or pharmaceutically acceptable salts, solvates thereof.

In one embodiment, the assay method comprises an antagonist compound of formula X is consisting compounds of general formula I-A pharmaceutically acceptable salts or solvates thereof:

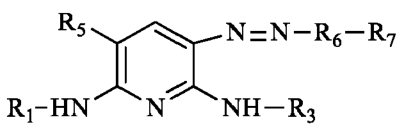

(Formula I-A)

wherein; $R_1$ and $R_3$ are the same or different and independently selected from hydrogen,

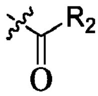 , 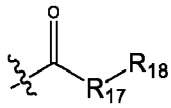 , and 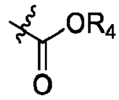 ;

wherein $R_2$ is selected from the group consisting of optionally substituted alkyl, alkenyl, alkynyl, cycloalkyl, heterocycloalkyl, aryl, or heteroaryl; $R_4$ is selected from the group consisting of optionally substituted alkyl, alkenyl, alkynyl, $-(CH_2-CH_2-O)_n-$, cycloalkyl, heterocycloalkyl, aryl, or heteroaryl, where n is from 1-20; $R_{17}$ is selected from optionally substituted linear or branched alkylene group, wherein alkylene is optionally substituted with amino, alkylamino and dialkylamino group; $R_{18}$ is selected from $NH_2$, $-NR_{20}R_{21}$, wherein $R_{20}$ and $R_{21}$ are each independently selected from hydrogen, optionally substituted alkyl, $-C(O)-R_{22}$, wherein $R_{22}$ is selected from optionally substituted alkyl, cycloalkyl, heterocycloalkyl, heteroaryl, and aryl group;

$R_5$ is selected from hydrogen, -OH, $-OR_8$, and

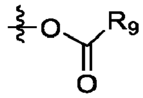 ;

wherein $R_9$ is selected from the group consisting of optionally substituted alkyl, alkenyl, alkynyl, cycloalkyl, heterocycloalkyl, aryl, heteroaryl, amino, alkyl-ester, alkoxy, heterocycloalkoxy, heteroaryloxy, cycloalkoxy, aryloxy, amino acid linked via ester or amide linkage at the point of attachment, $-(CH_2-CH_2-O)_n-$ , or $NR_{10}R_{11}$, where n is from 1-20; $R_{10}$ and $R_{11}$ are the same or different and independently selected from hydrogen or optionally substituted alkyl, cycloalkyl, aryl, heterocycloalkyl, and heteroaryl group; $R_8$ is selected from the group consisting of optionally substituted alkyl, alkenyl, alkynyl, cycloalkyl, heterocycloalkyl, aryl, or heteroaryl;

$R_6$ is a phenylene group, wherein the phenylene group is zero to four times substituted by $R_7$, wherein $R_7$ is selected from hydrogen, -OH, $-OR_{12}$,

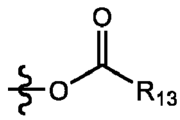

, 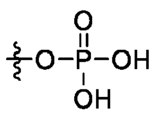 , 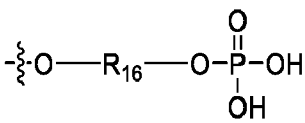 , 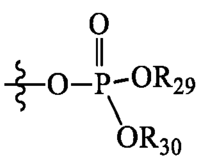

and $-R_{19}$; wherein $R_{12}$ is selected from the group consisting of optionally substituted alkyl, alkenyl, alkynyl, cycloalkyl, heterocycloalkyl, aryl, or heteroaryl; $R_{13}$ is selected from group consisting of optionally substituted alkyl, alkenyl, alkynyl, cycloalkyl, heterocycloalkyl, aryl, heteroaryl, alkyl-ester, amino, alkoxy, heterocycloalkoxy, heteroaryloxy, cycloalkoxy, aryloxy, aminoaryl, or amino acid linked via ester or amide linkage at the point of attachment, $-(CH_2-CH_2-O)_n-$, or $NR_{14}R_{15}$, where n is from 1-20; $R_{14}$ and $R_{15}$ are each independently selected from hydrogen, optionally substituted alkyl, heterocycloalkyl, aryl group; $R_{16}$ selected from optionally substituted alkylene$_{(C1-6)}$ group; $R_{19}$ is selected from $-O-R_{23}-O-C(O)-R_{24}$, $-O-C(O)-R_{23}-R_{27}$, and $-O-R_{23}-R_{28}$; wherein $R_{23}$ is optionally substituted linear or branched alkylene; $R_{24}$ is selected from optionally substituted alkoxy, cycloalkoxy, heterocycloalkoxy, heteroaryloxy, aryloxy group and $-N(R_{25}R_{26})$, wherein $R_{25}$, $R_{26}$ are each independently selected from hydrogen, optionally substituted alkyl and aryl, wherein alkyl and aryl are optionally substituted with OH, SH, F, Cl, Br, I, and optionally substituted hydroxyalkyl, amino group, or $R_{25}$ and $R_{26}$ is taken together to form an optionally substituted heterocycloalkyl ring, wherein the heterocycloalkyl ring is optionally substituted with alkyl, hydroxyalkyl, -OH, -SH, F, Cl, Br, I, and optionally substituted amino group;

$R_{27}$ is selected from optionally substituted alkyl, cycloalkyl, heterocycloalkyl, heteroaryl, aryl and -N($R_{25}R_{26}$) group;

$R_{28}$ is selected from optionally substituted alkyl, cycloalkyl, heterocycloalkyl, heteroaryl, and aryl group; wherein heterocycloalkyl, heteroaryl in $R_{28}$ is optionally substituted with alkyl, hydroxyalkyl group, OH, SH, F, Cl, Br, I, and optionally substituted, amino and oxo group;

$R_{29}$ and $R_{30}$ are each independently selected from hydrogen, and -$R_{23}$-O-C(O)-$OR_{31}$;wherein $R_{31}$ is selected from optionally substituted alkyl, cycloalkyl, heterocycloalkyl, aryl, and heteroaryl group.

## DETAILED DESCRIPTION OF THE INVENTION

The detailed description and the examples provided herein are exemplary and any modification or variation within the scope of the invention will be apparent to a person skilled in the art. Further, unless otherwise defined, all the technical and scientific terms used herein shall bear the meaning as understood by a person who is ordinarily skilled in the art.

As used herein, the term "IC50" is the half maximal inhibitory concentration **(IC50)** is a measure of the effectiveness of a substance in inhibiting a specific biological or biochemical function.

The term "site" refers to the possible therapeutic targets.

The term "about" as used herein, when referring to a measurable value is meant to encompass variations of $\pm$10%, preferably $\pm$5%, more preferably $\pm$1% and still more preferably $\pm$0.1% from the specified value.

The term "receptor of interest" any receptor that is present in human body which is expressed in cell preferably mammalian cell for in-vitro analysis.

In one embodiment the receptor of interest is G-protein-coupled receptors (GPCRs).

In one embodiment the G-protein-coupled receptors (GPCRs) receptor is bradykinin receptor.

The term "shaking" is interchangeable with "mixing".

The term "signal to noise ratio(SNR Or S/N)" is the ratio of the power of a signal (meaningful input) to the power of background noise (meaningless or unwanted input). "S/N" ratio is compares the level of a desired signal to the level of background noise. SNR is defined as the ratio of signal power to the noise power, often expressed in decibels. A ratio higher than 1:1 (greater than 0 dB) indicates more signal than noise.

The term "Z' value or Z' factor" is a measure of statistical effect size for use in high-throughput screening.

Formula used for calculating Z' value:

$$\text{Z-factor} = 1 - \frac{3(\sigma_p + \sigma_n)}{|\mu_p - \mu_n|}$$

The term "vehicle control group" means the group consist of suitable buffer and suitable solvent.

The term "working assay buffer (WAB)" means buffer having composition of assay buffer with 0.1% Bovine serum albumin and 2.5 mM probenecid.

The term "Calcium release" means to measure the calcium flux associated with Gq-protein coupled receptor activation or inhibition.

The term "Room temperature" refers to the temperature range from about $20^0$C to about $25^0$C.

In one embodiment the room temperature is selected form about $20^0$C, about $21^0$C, about $22^0$C, about $23^0$C, about $24^0$C, about $25^0$C. In other embodiment, Any ordinary person skilled in the art able to optimize temperature based on laboratory skill.

As used herein, the term "kinetic mode" is a mode of measurement in a kinetic method of analysis (time dependent manner), in which the concentration of a substance (e.g. Calcium by measuring fluorescence intensity) is determined from the rate of addition of a reagent (e.g. Agonist), as required to maintain a constant measured signal.

As used herein, the term "pharmaceutically acceptable" means salt, carriers, excipients, and other composition ingredients that are compatible with all other pharmaceutical ingredients of a composition and are not deleterious to an individual treated with composition.

As used herein, the term "solvate" is physical form of the compound I or a pharmaceutically acceptable salt thereof and either a stoichiometric or a non-stoichiometric amount of a solvent.

As used herein, the term "salt" refers to an acid or base salt of a compound of the invention. Salts of basic compounds are salts formed with mineral acids, organic carboxylic acids, organic sulfonic acids, and the like. Examples of suitable acids include hydrochloric, hydrobromic, sulfuric, nitric, perchloric, fumaric, maleic, phosphoric, glycollic, lactic, salicylic, succinic, toluene-p-Sulfonic, tartaric, acetic, citric, methanesulfonic, formic, benzoic, malonic, naphthalene-2-sulfonic, trifluoracetic acid and benzenesulfonic acids. Salts of acidic compounds are formed with bases, namely cationic species such as alkali and alkaline earth metal cations e.g., sodium, lithium, potassium, calcium, and magnesium ions, as well as ammonium cations e.g., ammonium, trimethylammonium and diethylammonium.

As used herein the term "baseline signal" or "baseline measurement" refers to a signal obtain without using any agonist which induces intracellular calcium release. Therefore baseline signal is only achieved in presence of antagonist which prevents or reduce intracellular calcium release. Baseline signal is useful in order to determine the competitive effect of antagonist and agonist thereby guide to skilled person to determine the potency of antagonist.

In an embodiment, the present invention provides an improved assay method to determine the efficacy of an antagonist by inhibiting the activity of bradykinin receptors.

In an embodiment, the present invention provides an improved assay method to determine the efficacy of an antagonist by inhibiting the activity of receptor of interest, the assay method comprising:

a. seeding a cell expressing the receptor of interest on the plate;
b. optionally incubating the cell for suitable time;
c. Washing the cell with suitable buffer selected from Hanks' Balanced Salt Solution (HBSS) and phosphate buffer; said buffer further comprising 1 M HEPES and optionally with probenecid;
d. Mixing the suitable dye to stain the cell;
e. Incubating the cell for suitable time;
f. Acclimatize the cell to suitable temperature for suitable time;
g. Washing the cell with suitable buffer;
h. Mixing the suitable antagonist diluted in suitable buffer;
i. Incubating and shaking the plate for suitable time at suitable temperature;
j. Reading the plate to determine the baseline signal;
k. mixing a suitable agonist diluted in suitable buffer;
l. Shaking the plate for suitable time;
m. Reading the plate to determine the receptor of interest inhibition activity of antagonist;
n. Wherein the assay improved the signal to noise ratio and lower the variability, in comparison to assay performed without washing.

In one embodiment, after performing the above method the IC50 values of antagonist selected from about 1 nM to about 20 $\mu$M.

In an embodiment, the present invention provides an improved assay method to determine the efficacy of an antagonist by inhibiting the activity of receptor of interest, the assay method comprising:

a. seeding a cell expressing the receptor of interest on the plate;
b. optionally incubating the cell for suitable time;
c. Washing the cell with suitable buffer selected from Hanks' Balanced Salt Solution (HBSS) and phosphate buffer; said buffer further comprising 1 M HEPES and optionally with probenecid;
d. Mixing the suitable dye to stain the cell;
e. Incubating the cell for suitable time;
f. Acclimatize the cell to suitable temperature for suitable time;
g. Mixing the suitable antagonist diluted in suitable buffer wherein the antagonist is selected from compound of formula X or pharmaceutically acceptable salts, solvates thereof;
h. Washing the cell with suitable buffer;
i. Mixing the suitable antagonist diluted in suitable buffer;
j. Incubating and shaking the plate for suitable time at suitable temperature;
k. Reading the plate to determine the baseline signal;
l. mixing a suitable agonist diluted in suitable buffer;
m. Shaking the plate for suitable time;
n. Reading the plate to determine the receptor of interest inhibition activity of antagonist;
o. Wherein the assay improved the signal to noise ratio and lower the variability, in comparison to assay performed without washing.

In one embodiment, after performing the above method the IC50 values of antagonist selected from about 1 nM to about 20 $\mu$M.

In one embodiment, cells used in present invention is mammalian cell.

In preferred embodiment, mammalian cell is Chinese Hamster Ovary cells (CHO). In most preferred embodiment, mammalian cell is Chinese Hamster Ovary cells-K1 (CHO-K1).

In one embodiment, cells expressing (CHO-K1) Bradykinin receptor are seeded on the plate, at seeding density from about 25,000 to about 40,000 cells/ well.

In one embodiment, cells expressing (CHO-K1) Bradykinin receptor are seeded on the plate, at seeding density preferably 30,000 cells/well.

In one embodiment, cells are incubated for 12-24hr in CO2 incubator at 37 °C and 5% CO2.

In one embodiment, cells are incubated for about 12 to about 24 hour in CO2 incubator at 37 °C and 5% CO2.

In one embodiment, cell culture media is removed, and cells are washed with about 150-about 200 μL assay buffer selected from Hanks'Balanced Salt Solution (HBSS) and phosphate buffer solution.

In one embodiment, cell culture media is removed, and cells are washed preferably with 150-200 μL assay buffer containing HBSS with 0.02 M HEPES.

In one embodiment, after washing, 100 μL dye solution is added to each well.

In one embodiment, suitable dye comprising Ex/Em = about 494nm to about 516nm.

In one embodiment, dye is selected from FLIPR Calcium 4, FLIPR Calcium 5, FLIPR Calcium 6 and 6-QF, Fluo-8 AM, fluo-4 , Fluo-4AM

In preferred embodiment, dye used in assay method is fluo-4 dye.

In one embodiment, suitable dye is fluorescent dye comprising Ex/Em = about 333nm to about 665nm.

In one embodiment, suitable dye is fluorescent dye comprising Ex/Em = about 400nm to about 665nm.

In one embodiment, the dye is non- limiting, any ordinary person skilled in the art able to optimize based on laboratory skill.

In one embodiment, dye is added into the assay plate and incubated for about 10 minutes to about 30 minutes.

In one embodiment, dye is added into the assay plate and incubated for about 10 minutes to about 30 minutes at suitable temperature selected from about $25^0$C to about $37^0$C.

In one embodiment, dye is added into the assay plate and for about 10 minutes at suitable temperature selected from about $37^0$C.

Post incubation, the assay plate is acclimatized to suitable temperature selected from 20 $^0$C to 25 $^0$C. In preferred embodiment the suitable temperature is 25°C.

In certain embodiment the acclimatization is performed for about 5 min to about 35 min. In an embodiment the acclimatization is performed from about 10 min to about 30 min. In an embodiment the acclimatization is performed for about 30 min.

In one embodiment, current method comprises the washing step to remove extra dye or before adding working assay buffer.

In one embodiment, washing improves the efficacy of assay by improving signal to noise ratio.

In one embodiment, washing surprisingly improving the response of either alone or both agonist and antagonist.

In one embodiment, washing reduces the variation withing the group of same concentration of compound. In other embodiment, washing reduces the variation between the replicates of same concentration of compound.

In one embodiment, the washing improves the difference between the vehicle control group and the group containing suitable compound.

In one embodiment, washing reduces the variation withing the group of same concentration for compound selected from compound I, compound II, compound III, compound IV, compound V. However, skilled person can be used any compound to determine it's antagonistic potency through the present assay method. In one embodiment, the present invention shows that by incorporating washing step in the protocol, higher values of fluorescence obtained which makes the window broader and hence helps to determine percent inhibition by the compounds accurately.

In one embodiment, the present invention shows that by incorporating washing step in the protocol gives higher values of fluorescence are selected from about 60,000 RFU to about 210000 RFU(relative fluorescence unit).

In one embodiment, the present invention shows that by incorporating washing step in the protocol gives higher values of fluorescence are selected from about 60,000 RFU, about 70,000 RFU, about 80,000 RFU, about 90,000 RFU, about 100000 RFU, about 110,000 RFU, about 120,000 RFU, about 130,000 RFU, about 140,000 RFU, about 150,000 RFU, about 160,000 RFU, about 170,000 RFU, about 180,000 RFU, about 190,000 RFU, about 200000 RFU, about 210000 RFU.

In one embodiment the washing step performed during assay improves signal to noise ratio.

In one embodiment, the washing provides the higher Z' values.

In one embodiment, the washing provides the higher Z' values selected from about 0.3, about 0.4, about 0.5, about 0.6, about 0.7, about 0.8, about 0.9, about 1.0. In preferred embodiment the washing provides the higher Z' values selected from about 0.5, about 0.6, about 0.7, about 0.8, about 0.9, about 1.0. In preferred embodiment the washing provides the higher Z' values selected from about 0.8, about 0.9, about 1.0.

In one embodiment, washing is performed by working assay buffer (WAB) in suitable concentration selected from 1 μL, 10 μL, 20 μL, 30 μL, 40 μL, 50 μL, 60 μL, 70 μL, 80 μL, 90 μL, 100 μL, 110 μL, 120 μL, 130 μL, 140 μL, 150 μL, 160 μL, 170 μL, 180 μL, 190 μL, 200 μL, 210 μL, 220 μL, 230 μL, 240 μL, 250 μL, 260 μL, 270 μL, 280 μL,

290 μL, 300 μL having composition assay buffer with 0.1% Bovine serum albumin and 2.5 mM probenecid.

In one embodiment, washing is performed by working assay buffer (WAB) in suitable concentration selected from 100 μL to 300 μL. In one embodiment, washing is performed by working assay buffer (WAB) in suitable concentration selected from 150 μL to 250 μL. In one embodiment, washing is performed by working assay buffer (WAB) in suitable concentration selected from 150 μL to 200 μL.

In one embodiment, add 50 μL antagonist dilution or 50 μL standard antagonist concentration >IC50 concentration is prepared in WAB or 50 μL WAB as antagonist replacement.

In one embodiment, add 50 μL antagonist dilution of standard antagonist concentration more than IC50 prepared in WAB or add 50 μL WAB as antagonist replacement.

The assay plates are subjected to incubation post addition of antagonist for suitable time period selected from about 5min, about 10 min, about 15 min, about 20 min, about 25 min, about 30 min and about 35 min. In certain embodiment the assay plates are subjected to incubation post addition of antagonist for suitable time period selected from about 15 min to about 30 min.

In one embodiment, incubate the plate for about 10 to about 30 min at room temperature with shaking at about 200 to about 500 rpm.

In one embodiment, incubate the plate preferably for 20 min at room temperature with shaking preferably at 300 rpm.

In one embodiment, read the plate in kinetic mode (from 30sec to 1 minute time frame), Ex/Em = 494/516 nm for baseline measurement.

In present method, the agonist is added in the assay plate to determine the cell response which can be measured through calcium release. In certain embodiment the agonist is added in suitable volume from about 10 μL to about 100 μL. In an embodiment the volume of agonist is 50 μL. In an embodiment the agonist volume is similar to volume of antagonist. Any skilled person can determine the suitable volume of agonist based on the volume of antagonist used in the assay plate.

In one embodiment, add 50 μL agonist dilutions, approximately of EC80 concentration or 50 μL positive control 0.1% triton X prepared in WAB.

In one embodiment, add 50 μL agonist dilutions, approximately of EC80 concentration or 50 μL positive control 0.1% triton X prepared in suitable buffer.

In one embodiment, the suitable buffer is washing assay buffer and the buffer is similar to the assay buffer. In one embodiment, Shake the plate manually/using shaker for 5 to10 seconds for proper mixing.

In one embodiment, read the plate in kinetic mode for another 30 to 120 seconds.

In one embodiment, the plate reading is performed at least in about 90 seconds to about 5 minutes.

In one embodiment, the plate reading is performed at least in about 90 seconds to about 10 minutes.

In one embodiment, read the plate in kinetic mode for another about 90 seconds to about 10 minutes.

In one embodiment, read the plate in kinetic mode for another about 1 minute, about 2 minute, about 3 minute, about 4 minute, about 5 minute, about 6 minute, about 7 minute, about 8 minute, about 9 minute, about 10 minutes.

In one embodiment, a suitable agonist and antagonist selected from:

| Bradykinin receptor | Agoni st | Antagonist |
|---|---|---|
| B1 | LDBK | R715 |
| B2 | BK | HOE140 |

In one embodiment, IC50 values of antagonist selected from 1 nM to 20 μM.

In one embodiment, IC50 values of antagonist selected from about 1 nM to about 20 μM.

In one embodiment, IC50 values of antagonist selected from, 1 nM to 20 μM, 1 nM to 15 μM, InM to 10 μM, 1 nM to 5 μM, 1 nM to 1 μM, 1 nM to 0.5 μM, and 1 nM to 0.1 μM.

In one embodiment, IC50 values of antagonist selected from, about a1 nM about to 20 μM, about 1 nM to about 15 μM, about InM to about 10 μM, about 1 nM to about 5 μM, about a1 nM to about 1 μM, about 1 nM to about 0.5 μM, and about 1 nM to about 0.1 μM.

In another embodiment, the assay method comprises a one or more antagonist consisting of formula X. In other embodiment formula X consist of formula I and I-A.

In another embodiment, the assay method comprises a one or more antagonist selected from the group consisting of;

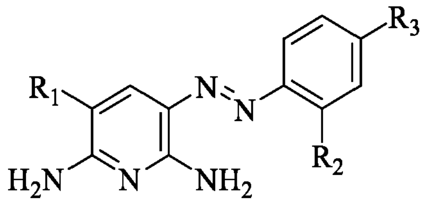

Formula I

; and pharmaceutically acceptable salts, solvates thereof; wherein $R_1$, $R_2$ and $R_3$ can be selected independently from hydrogen and hydroxyl group.

In another embodiment, the assay method comprises a one or more antagonist selected from the group consisting of;

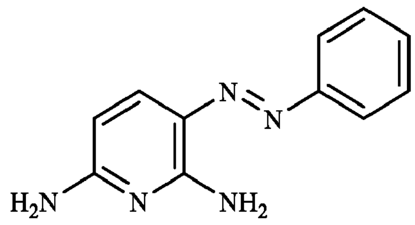

Compound I ,

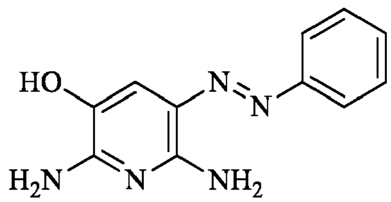

Compound II ,

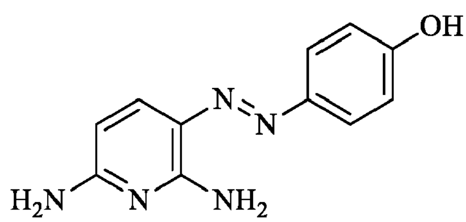

Compound III ,

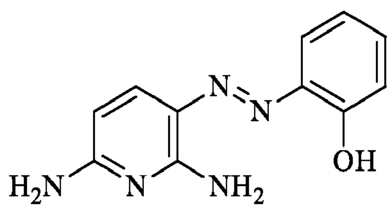

Compound IV ,

and

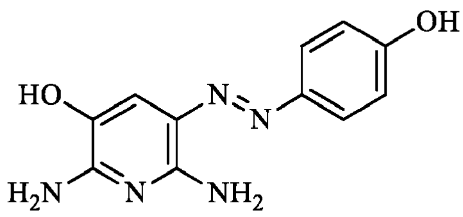

Compound V

;

or pharmaceutically acceptable salts, solvates thereof.

In one embodiment, the assay method comprises an antagonist compound of formula X is consisting compounds of general formula I-A pharmaceutically acceptable salts or solvates thereof:

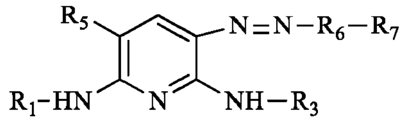

(Formula I-A)

wherein; $R_1$ and $R_3$ are the same or different and independently selected from hydrogen,

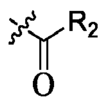 , 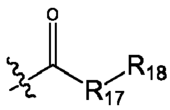 , and 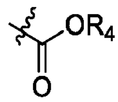 ;

wherein $R_2$ is selected from the group consisting of optionally substituted alkyl, alkenyl, alkynyl, cycloalkyl, heterocycloalkyl, aryl, or heteroaryl; $R_4$ is selected from the group consisting of optionally substituted alkyl, alkenyl, alkynyl, $-(CH_2-CH_2-O)_n-$, cycloalkyl, heterocycloalkyl, aryl, or heteroaryl, where n is from 1-20; $R_{17}$ is selected from optionally substituted linear or branched alkylene group, wherein alkylene is optionally substituted with amino, alkylamino and dialkylamino group; $R_{18}$ is selected from $NH_2$, $-NR_{20}R_{21}$, wherein $R_{20}$ and $R_{21}$ are each independently selected from hydrogen, optionally substituted alkyl, $-C(O)-R_{22}$, wherein $R_{22}$ is selected from optionally substituted alkyl, cycloalkyl, heterocycloalkyl, heteroaryl, and aryl group;

$R_5$ is selected from hydrogen, -OH, $-OR_8$, and

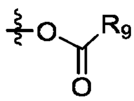

;

wherein $R_9$ is selected from the group consisting of optionally substituted alkyl, alkenyl, alkynyl, cycloalkyl, heterocycloalkyl, aryl, heteroaryl, amino, alkyl-ester, alkoxy, heterocycloalkoxy, heteroaryloxy, cycloalkoxy, aryloxy, amino acid linked via ester or amide linkage at the point of attachment, $-(CH_2-CH_2-O)_n-$ , or $NR_{10}R_{11}$, where n is from 1-20; $R_{10}$ and $R_{11}$ are the same or different and independently selected from hydrogen or optionally substituted alkyl, cycloalkyl, aryl, heterocycloalkyl, and heteroaryl group; $R_8$ is selected from the group consisting of optionally substituted alkyl, alkenyl, alkynyl, cycloalkyl, heterocycloalkyl, aryl, or heteroaryl;

$R_6$ is a phenylene group, wherein the phenylene group is zero to four times substituted by $R_7$, wherein $R_7$ is selected from hydrogen, -OH, $-OR_{12}$,

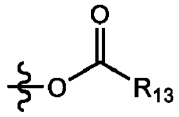

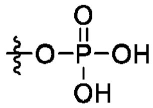, 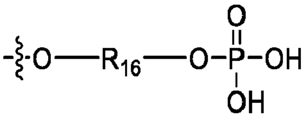, 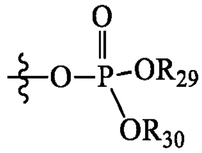,

and - $R_{19}$; wherein $R_{12}$ is selected from the group consisting of optionally substituted alkyl, alkenyl, alkynyl, cycloalkyl, heterocycloalkyl, aryl, or heteroaryl; $R_{13}$ is selected from group consisting of optionally substituted alkyl, alkenyl, alkynyl, cycloalkyl, heterocycloalkyl, aryl, heteroaryl, alkyl-ester, amino, alkoxy, heterocycloalkoxy, heteroaryloxy, cycloalkoxy, aryloxy, aminoaryl, or amino acid linked via ester or amide linkage at the point of attachment, $-(CH_2-CH_2-O)_n-$, or $NR_{14}R_{15}$, where n is from 1-20; $R_{14}$ and $R_{15}$ are each independently selected from hydrogen, optionally substituted alkyl, heterocycloalkyl, aryl group; $R_{16}$ selected from optionally substituted alkylene$_{(C1-6)}$ group; $R_{19}$ is selected from $-O-R_{23}-O-C(O)-R_{24}$, $-O-C(O)-R_{23}-R_{27}$, and $-O-R_{23}-R_{28}$; wherein $R_{23}$ is optionally substituted linear or branched alkylene; $R_{24}$ is selected from optionally substituted alkoxy, cycloalkoxy, heterocycloalkoxy, heteroaryloxy, aryloxy group and $-N(R_{25}R_{26})$, wherein $R_{25}$, $R_{26}$ are each independently selected from hydrogen, optionally substituted alkyl and aryl, wherein alkyl and aryl are optionally substituted with OH, SH, F, Cl, Br, I, and optionally substituted hydroxyalkyl, amino group, or $R_{25}$ and $R_{26}$ is taken together to form an optionally substituted heterocycloalkyl ring, wherein the heterocycloalkyl ring is optionally substituted with alkyl, hydroxyalkyl, -OH, -SH, F, Cl, Br, I, and optionally substituted amino group;

$R_{27}$ is selected from optionally substituted alkyl, cycloalkyl, heterocycloalkyl, heteroaryl, aryl and $-N(R_{25}R_{26})$ group;

$R_{28}$ is selected from optionally substituted alkyl, cycloalkyl, heterocycloalkyl, heteroaryl, and aryl group; wherein heterocycloalkyl, heteroaryl in $R_{28}$ is optionally substituted with alkyl, hydroxyalkyl group, OH, SH, F, Cl, Br, I, and optionally substituted, amino and oxo group;

$R_{29}$ and $R_{30}$ are each independently selected from hydrogen, and $-R_{23}-O-C(O)-OR_{31}$;wherein $R_{31}$ is selected from optionally substituted alkyl, cycloalkyl, heterocycloalkyl, aryl, and heteroaryl group.

In a such embodiment, the assay method is useful in determining the antagonistic effect of desired compound by inhibiting the activity of GPCR wherein the compounds are useful in the treatment of diseases including but not limited to interstitial cystitis/bladder pain syndrome (IC/BPS), over active bladder (OAB), chronic cough, endometriosis, neuropathic pain, migraine, pruritus, postherpetic neuralgia, urinary tract infection related pain, osteoarthritis, arthritis , IC/BPS with or without Hunner's lesion, urologic chronic pelvic pain syndrome (UCPPS), chronic prostatitis, urinary tract infection (UTI) induced pelvic pain, cancer pain syndromes of the genitourinary region, urogenital pain syndromes, vulvodynia, pelvic floor dysfunction, urethral syndrome and urinary incontinence.

In a such embodiment, , the assay method is useful in determining the antagonistic effect of desired compound of formula I, Formula I-A, by inhibiting the activity of bradykinin receptors wherein the compounds are useful in the treatment of interstitial cystitis/bladder pain syndrome (IC/BPS), over active bladder (OAB), chronic cough, endometriosis, neuropathic pain, migraine, pruritus, postherpetic neuralgia, urinary tract infection related pain, osteoarthritis, arthritis , IC/BPS with or without Hunner's lesion, urologic chronic pelvic pain syndrome (UCPPS), chronic prostatitis, urinary tract infection (UTI) induced pelvic pain, cancer pain syndromes of the genitourinary region, urogenital pain syndromes, vulvodynia, pelvic floor dysfunction, urethral syndrome and urinary incontinence.

In one embodiment, the assay method is useful in determining the antagonistic effect of desired compound by inhibiting the activity of bradykinin receptors useful in the prevention or treatment of urinary bladder pain; wherein the method comprising;

a. seeding a cell expressing the receptor of interest on the plate;
b. optionally incubating the cell for suitable time;
c. Washing the cell with suitable buffer selected from Hanks' Balanced Salt Solution (HBSS) and phosphate buffer; said buffer further comprising 1 M HEPES and optionally with probenecid;
d. Mixing the suitable dye to stain the cell;

e. Incubating the cell for suitable time;
f. Acclimatize the cell to suitable temperature for suitable time;
g. Washing the cell with suitable buffer;
h. Mixing the suitable antagonist diluted in suitable buffer;
i. Incubating and shaking the plate for suitable time at suitable temperature;
j. Reading the plate to determine the baseline signal;
k. mixing a suitable agonist diluted in suitable buffer;
l. Shaking the plate for suitable time;
m. Reading the plate to determine the receptor of interest inhibition activity of antagonist;
n. Wherein the assay improved the signal to noise ratio and lower the variability, in comparison to assay performed without washing.

In one embodiment, after performing the above method the IC50 values of antagonist selected from about 1 nM to about 20 μM.

The present invention provides the following examples for illustrative purpose only and scope of the invention should not be considered limited to them.

## Experimental Details

### Materials:

Human bradykinin B1 receptor cell line (Product#ES-091-C), Human bradykinin B2 receptor aequorin cell line (Product# ES-090-A), Fluo-4 calcium assay kits (Molecular Probes).

Agonist for B1 receptor cell line is Lys-[Des-Arg9]-Bradykinin (LDBK) and antagonist is R715. Agonist for B2 receptor cell line is BK (bradykinin) and antagonist is HOE140. All the agonist and antagonist were procured from TOCRIS. Dye loading solution was prepared by adding 10 mL of assay buffer and 100 μL of the probenecid stock solution (provided in the Kit) to one bottle of component A (dye concentrate). 0.4% Triton X-100 (4X) was used as positive control (PC) and was prepared by adding 38 uL of Triton X-100 in 10 mL of working assay buffer (WAB).

### Cell Culture and Treatment:

Human bradykinin B1 receptor cell line (B1) and human bradykinin B2 receptor aequorin cell line (B2) was procured from PerkinElmer, Waltham, MA. B1 cells were cultured and maintained in Ham's F-12 medium (Gibco) supplemented with 10% fetal bovine serum (FBS) (Gibco) and 0.4 mg/ml Geneticin (Gibco) in a humidified incubator at 37°C with 5% CO2. B2 cells were cultured and maintained in Ham's F-12 medium (Gibco) supplemented with 10% fetal bovine serum (FBS) (Gibco), 0.4 mg/ml Geneticin (Gibco) and 5 μg/ml Puromycin (Gibco) in a humidified incubator at 37 °C with 5% CO2. All the test compounds were weighed and dissolved in DMSO and a stock of 20 mM was prepared and stored at -20 °C.

### Methodology for Bradykinin Receptor Antagonist Screening Assay:

Bradykinin receptor antagonist screening assay was performed using Fluo-4 calcium assay kits by following the manufacturer's instruction. Briefly, approximately 30,000 of human either B1 or B2 cells were seeded in each well of a 96 black well plate. After 24 h, the growth media was removed and washed with 200 μL of assay buffer provided in the Fluo-4 calcium assay kits. Thereafter, 100 uL of Flu-4 dye-loading solution was added in each well of 96 well plate and incubated it at 37 °C for 30 min. After 30 minutes of incubation at room temperature, dye loading solution was removed and washed with 200 μL of WAB (mentioned in materials). Post washing, into 100 uL of WAB, 50 uL of WAB or 50 uL of cell line receptor specific antagonist (mentioned in materials) or test compound dilutions were added in 96 well plate. The plates were incubated for 20 min at room temperature with shaking at 300 rpm. At the end of the incubation time, the plates were inserted into the CLARIOstar microplate reader and %gain was adjusted. Thereafter, the plates were read at Ex/Em = 494/516 nm for baseline RFU (Range 1). Thereafter, the microplate reader was paused at $11^{th}$ cycle and 50 uL of 4x (EC80) cell line specific agonist (mentioned in materials) or PC dilution prepared in WAB was added and manually the plates were shaked for 5 sec. Again, the plates were read at Ex/Em = 494/516 nm (Range 2). Delta RFU was calculated using the formula:

$$\text{Delta RFU} = \text{Range 2 avg RFU} - \text{Range 1 avg RFU}$$

Based on the data the IC50 or Percent Inhibition of all the compounds was calculated.

Note: The above assay was performed using plate (flying) mode function of CLARIOstar.

The following examples are given for the purpose of illustrating the present invention and should not be considered as limiting the scope of the invention.

**Example 1: Calcium mobilization Bioassay for Bradykinin B1 and B2 Receptor compounds**

| **Sr. no.** | **Compound ID** | **Bradykinin B1 receptor Antagonistic activity by Intracellular calcium assay** | **Bradykinin B2 receptor Antagonistic activity by Intracellular calcium assay** |
|---|---|---|---|
| | | **IC50 (μM)** | **IC50 (μM)** |
| 1. | Compound I | 32.4 | 31.9 |
| 2. | Compound II | 12.5 | 13.2 |
| 3. | Compound III | 27.1 | 21.3 |
| 4. | Compound IV | 8.4 | 10.9 |
| 5. | Compound V | 29.5 | >100 |

**Example 2: Calcium mobilization Bioassay for Bradykinin Receptor compounds and role of addition of washing step in the protocol**

| **Compound** | **$IC_{50}$ obtained without washing step** | **$IC_{50}$ obtained with washing step (Mean ± SEM)** |
|---|---|---|
| HOE-140 | 52.728 nM | 2.99 ± 1.35 nM |

As evident from table mentioned in Example 2, when experiment was performed without washing steps, we found the $IC_{50}$ of HOE-140 was approximately 15-fold higher than the $IC_{50}$ obtained by incorporating washing step in the assay method.

**Example 3: Assay to provide reduced variation and improved fluorescence**

The below mentioned table shows that there was no difference found between the fluorescence values in the replicates having same compound concentration of compound treated group. Also, the below table shows that by incorporating washing step in the protocol, higher values of fluorescence obtained which gives a broader range of RFU values between the groups.

| Group | RFU value | | | Average RFU value | Positive control to negative control ratio |
|---|---|---|---|---|---|
| **With wash** | | | | | |
| Compound with a given concentration in 0.24μM | 61715.7 | 68821.2 | 63455.15 | 64664 | 257 |
| Positive control | 176211.95 | 137719.35 | 150239.2 | 154723 | |
| 0.1% TritonX 100 | | | | | |
| Negative control (WAB) | 479.85 | 1185.85 | 138.9 | 601.5 | |

(continued)

| Without wash | | | | | |
|---|---|---|---|---|---|
| Compound with a given concentration in 0.24 μM | 28296 | 23788 | 26863 | 26315.7 | 1.6 |
| Positive control 0.1% TritonX 100 | 40936 | 45351 | - | 43143.5 | |
| Negative control (WAB) | 24020 | 28287 | - | 26153.5 | |

As evident from table mentioned in Example 3, when there was no washing step incorporated in the protocol, there was less difference found in RFU values between compound treated group and negative control group. Due to the less difference in RFU values it is difficult for person skilled in the art to find out the actual inhibition by the compound. When experiment was performed with incorporation of washing step in the protocol, we found significant difference between positive control and negative control. Also, difference in RFU values between negative control group and Compound treated groups, which helps in differentiating the inhibition activity of compound.

**Example 4: Determination of Z' value**

By referring the experiment of Example 2, the calculated Z' value is shown in the below table:

| Group | RFU values | Mean | Std. deviation | Z' value |
|---|---|---|---|---|
| Positive control | 210536 | 209134.5 | 1982.020308 | 0.99 |
| | 207733 | | | |
| Negative control | 3501 | 3006 | 700.0357134 | |
| | 2511 | | | |

Formula used for calculating Z' value:

$$\text{Z-factor} = 1 - \frac{3(\sigma_p + \sigma_n)}{|\mu_p - \mu_n|}$$

Wherein, σ is the standard deviation of positive control (p) and negative control (n) and μ is the mean of positive control (p) and negative control (n).

**Claims**

**1.** An improved assay method to determine the efficacy of an antagonist by inhibiting the activity of bradykinin receptors; the assay method comprising:

a. seeding a cell expressing the receptor of interest on the plate;
b. optionally incubating the cell for suitable time;
c. Washing the cell with suitable buffer selected from Hanks' Balanced Salt Solution (HBSS) and phosphate buffer; said buffer further comprising 1 M HEPES and optionally with probenecid;
d. Mixing the suitable dye to stain the cell;
e. Incubating the cell for suitable time;
f. Acclimatize the cell to suitable temperature for suitable time;
g. Washing the cell with suitable buffer;
h. Mixing the suitable antagonist diluted in suitable buffer;
i. Incubating and shaking the plate for suitable time at suitable temperature;
j. Reading the plate to determine the baseline signal;
k. mixing a suitable agonist diluted in suitable buffer;

l. Shaking the plate for suitable time;
m. Reading the plate to determine the receptor of interest inhibition activity of antagonist;
n. Wherein the assay improved the signal to noise ratio and lower the variability, in comparison to assay performed without washing.

**2.** The assay method according to claim 1 wherein the IC50 values of antagonist selected from about 1 nM to about 20 μM.

**3.** The method according to claim 1 wherein the, IC50 values of antagonist selected from, about 1 nM about to 20 μM, about 1 nM to about 15 μM, about 1nM to about 10 μM, about 1 nM to about 5 μM, about a1 nM to about 1 μM, about 1 nM to about 0.5 μM, and about 1 nM to about 0.1 μM.

**4.** The assay method according to claim 1, wherein the mammalian cell is Chinese Hamster Ovary cells (CHO).

**5.** The assay method according to claim 1, wherein the mammalian cell used are seeded on the plate at seeding density from about 25,000 to about 40,000 cells/ well.

**6.** The mammalian cell used in the assay method according to claim 5, at seeding density from 30,000 cells/ well.

**7.** The assay method according to claim 1, wherein the incubation is performed for about 12 to about 24 hours in $CO_2$ incubator at 37 °C and 5% $CO_2$.

**8.** The assay method according to claim 1, wherein the washing is performed with about 150 to about 200 μL assay buffer.

**9.** The assay method according to claim 1, wherein the suitable dye is fluorescent dye comprising Ex/Em = about 333nm to about 665nm.

**10.** The assay method according to claim 9, wherein the suitable dye comprising Ex/Em = about 400nm to about 665nm.

**11.** The assay method according to claim 10, wherein the suitable dye comprising Ex/Em = about 494nm to about 516nm.

**12.** The assay method according to claim 1 wherein the suitable dye is selected from FLIPR Calcium 4, FLIPR Calcium 5, FLIPR Calcium 6 and 6-QF, Fluo-8 AM, fluo-4 NW, Fluo-4AM

**13.** The assay method according to claim 1 wherein the incubation is performed in step (e) is about 20 minutes to about 40 minutes.

**14.** The assay method according to claim 1 wherein the acclimatization is performed in step (f) is at suitable temperature from 20°C to 25°C.

**15.** The assay method according to claim 1 wherein the antagonist is selected from compound of formula X.

**16.** The assay method according to claim 1 wherein the incubation is performed in step (i) is about 10 minutes to about 30 minutes at suitable temperature selected from 25°C to about 37°C.

**17.** The assay method according to claim 16 wherein the incubation is performed in step (i) is about 10 minutes at suitable temperature selected from 37°C.

**18.** The assay method according to claim 1 wherein the agonist is selected from Lys-[Des-Arg9]-Bradykinin (LDBK), and BK (bradykinin) for Bradykinin B1 receptor and Bradykinin B2 receptor respectively.

**19.** The assay method according to claim 1 wherein the suitable buffer in step (l) is similar to buffer used in step (c).

**20.** The assay method according to claim 1 wherein the receptor of interest in step (n) is GPCR receptor.

**21.** The assay method according to claim 20 wherein the receptor of interest in step (n) is bradykinin receptor.

**22.** The assay method according to claim 1 wherein the plate reading is performed at least in about 90 seconds to 10 minutes.

**23.** The assay method according to claim 22 wherein the plate reading is performed at least in about 90 seconds to 5 minutes.

**24.** The assay method according to claim 23 wherein the plate reading is performed at least in about 90 seconds.

**25.** The method according to claim 1, wherein washing improving the response of agonist and antagonist.

**26.** The method according to claim 1, wherein washing reduces the variation withing the group of same concentration of compound.

**27.** The method according to claim 1, wherein washing improves the difference between the vehicle control group and the group containing suitable compound.

**28.** The method according to claim 1, wherein washing reduces the variation withing the group of same concentration for compound selected from the compound I, compound II, compound III, compound IV, compound V.

**29.** The method according to claim 1, wherein washing step provides the higher values of fluorescence.

**30.** The method according to claim 30, wherein washing step gives higher values of fluorescence selected from about 60,000 RFU to about 210000 RFU.

**31.** The method according to claim 1, wherein the washing provides the higher Z' values selected from about 0.3, about 0.4, about 0.5, about 0.6, about 0.7, about 0.8, about 0.9, about 1.0.

**32.** The method according to claim 1, wherein the assay method comprises a one or more antagonist selected from the group consisting of; the compound of formula X is consisting of general formula I;

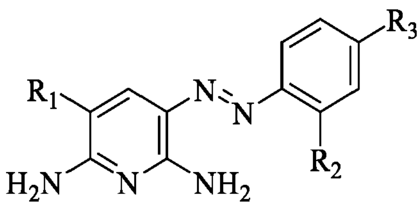

Formula I

; and pharmaceutically acceptable salts, solvates thereof wherein $R_1$, $R_2$ and $R_3$ can be selected independently from hydrogen and hydroxyl group.

**33.** The compound according to claim 32,the assay method comprises a one or more antagonist selected from the group consisting of;

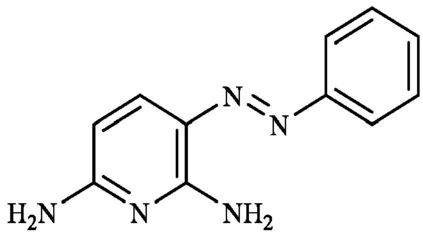

Compound I

,

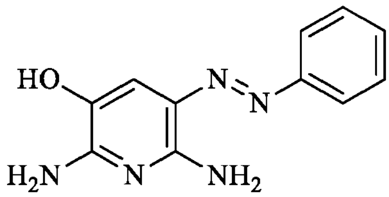

Compound II ,

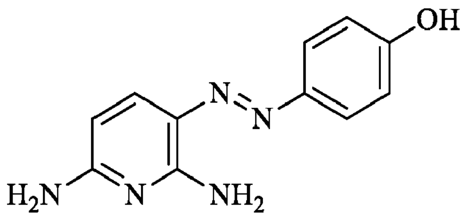

Compound III ,

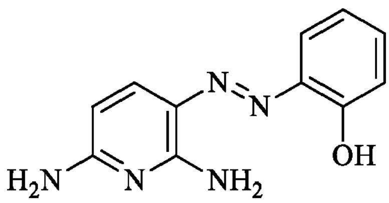

Compound IV ,

and

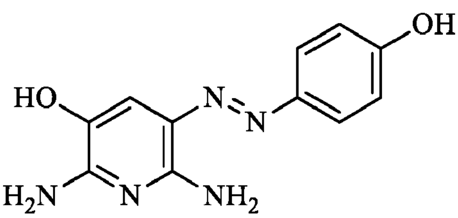

Compound V ;

or pharmaceutically acceptable salts, solvates thereof.

**34.** The assay method is useful in determining the antagonistic effect of desired compound of formula I, Formula I-A, by inhibiting the activity of bradykinin receptors wherein the compounds are useful in the treatment of interstitial cystitis/bladder pain syndrome (IC/BPS), over active bladder (OAB), chronic cough, endometriosis, neuropathic pain, migraine, pruritus, postherpetic neuralgia, urinary tract infection related pain, osteoarthritis, arthritis, IC/BPS with or without Hunner's lesion, urologic chronic pelvic pain syndrome (UCPPS), chronic prostatitis, urinary tract infection (UTI) induced pelvic pain, cancer pain syndromes of the genitourinary region, urogenital pain syndromes, vulvodynia, pelvic floor dysfunction, urethral syndrome and urinary incontinence.

Europäisches Patentamt
European Patent Office
Office européen des brevets

# EUROPEAN SEARCH REPORT

Application Number
EP 22 16 0038

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2005/042027 A2 (AMGEN INC [US]; NG GORDON [US] ET AL.) 12 May 2005 (2005-05-12) * p.93 * | 1-15,18, 20,25, 27,31-34 | INV. G01N33/68 |
| X | US 2004/009537 A1 (ROOS JACK [US] ET AL) 15 January 2004 (2004-01-15) * paragraph [0469] - paragraph [0469] * | 1-15,18, 20,25, 27,31-34 | |
| X | RANSOM R W ET AL: "Pharmacological characterization and radioligand binding properties of a high-affinity, nonpeptide, bradykinin B"1 receptor antagonist", EUROPEAN JOURNAL OF PHARMACOLOGY, ELSEVIER SCIENCE, NL, vol. 499, no. 1-2, 19 September 2004 (2004-09-19), pages 77-84, XP004567149, ISSN: 0014-2999, DOI: 10.1016/J.EJPHAR.2004.07.104 * abstract, materials and methods: 2.3.3 Calcium mobilization * | 1-15,18, 20,25, 27,31-34 | TECHNICAL FIELDS SEARCHED (IPC): G01N |
| X | DINESH SRINIVASAN ET AL: "Pharmacological characterization of canine bradykinin receptors in prostatic culture and in isolated prostate", BRITISH JOURNAL OF PHARMACOLOGY, WILEY-BLACKWELL, UK, vol. 142, no. 2, 29 January 2009 (2009-01-29), pages 297-304, XP071130065, ISSN: 0007-1188, DOI: 10.1038/SJ.BJP.0705757 * abstract; materials and methods: Measurement of intracellular calcium using the fluorometric imaging plate reader (FLIPR) * | 1-15,18, 20,25, 27,31-34 | |

The present search report has been drawn up for all claims

1

| Place of search | Date of completion of the search | Examiner |
|---|---|---|
| Munich | 16 November 2022 | Behrens, Ralf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 16 0038

This annex lists the patent family members relating to the patent documents cited in the above-mentioned European search report. The members are as contained in the European Patent Office EDP file on
The European Patent Office is in no way liable for these particulars which are merely given for the purpose of information.

16-11-2022

| Patent document cited in search report | Publication date | Patent family member(s) | Publication date |
|---|---|---|---|
| WO 2005042027 A2 | 12-05-2005 | AU 2004285467 A1 | 12-05-2005 |
| | | BR PI0415755 A | 19-12-2006 |
| | | CA 2545499 A1 | 12-05-2005 |
| | | EA 200600817 A1 | 29-12-2006 |
| | | EC SP066575 A | 17-10-2006 |
| | | EP 1677830 A2 | 12-07-2006 |
| | | ES 2395546 T3 | 13-02-2013 |
| | | JP 4896728 B2 | 14-03-2012 |
| | | JP 2007526240 A | 13-09-2007 |
| | | KR 20060105757 A | 11-10-2006 |
| | | KR 20080015054 A | 15-02-2008 |
| | | MA 28160 A1 | 01-09-2006 |
| | | SG 149872 A1 | 27-02-2009 |
| | | US 2005215470 A1 | 29-09-2005 |
| | | US 2009048149 A1 | 19-02-2009 |
| | | US 2009137452 A1 | 28-05-2009 |
| | | US 2009137483 A1 | 28-05-2009 |
| | | WO 2005042027 A2 | 12-05-2005 |
| US 2004009537 A1 | 15-01-2004 | NONE | |

EPO FORM P0459

For more details about this annex : see Official Journal of the European Patent Office, No. 12/82